(19) **Europäisches Patentamt**
**European Patent Office**
**Office européen des brevets**

(11) **EP 4 672 139 A1**

(12) **EUROPEAN PATENT APPLICATION**

(43) Date of publication:
**31.12.2025 Bulletin 2026/01**

(21) Application number: **25184837.0**

(22) Date of filing: **24.06.2025**

(51) International Patent Classification (IPC):
**G06T 5/73** (2024.01)   **G06T 7/55** (2017.01)

(52) Cooperative Patent Classification (CPC):
**G06T 7/55; G06T 5/73;** G06T 2207/10028;
G06T 2207/30036; G06T 2210/41

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **24.06.2024 DK PA202470177**

(71) Applicant: **3Shape A/S
1060 Copenhagen K (DK)**

(72) Inventors:
• **DRAGUNOV, Denys
1060 Copenhagen K (DK)**
• **HOEDT, Asger Vejen
1060 Copenhagen K (DK)**
• **BONDORF, Johan Teichert
1060 Copenhagen K (DK)**

(74) Representative: **Guardian
IP Consulting I/S
Diplomvej, Building 381
2800 Kgs. Lyngby (DK)**

(54) **A SYSTEM AND METHOD FOR OPTIMIZING A THREE-DIMENSIONAL MODEL OF A DENTAL OBJECT**

(57)   The present disclosure relates to an intraoral scanner system and a method for optimizing a 3D model for motion blur and warp. The method may comprise receiving a 3D model that is determined by averaging a plurality of intraoral scans of a dental object captured by an intraoral scanner, wherein each of the plurality of intraoral scans includes a plurality of 3D scan points, determining a plurality of corresponding sample 3D scan points of the 3D model by selecting each of the plurality of corresponding sample 3D scan points in the 3D model that is closest to a 3D scan point of the identified plurality of 3D scan points, determining, based on a plurality of transformation matrices, a plurality of adjusted 3D scan points for each of the plurality of identified 3D scan points, by minimizing a distance between each of the plurality of identified 3D scan points relative to each of the plurality of corresponding sample 3D scan points for each of the plurality of transformation matrices, assigning different weighting coefficients to the plurality of adjusted 3D scan points for each of the plurality of identified 3D scan points, and optimizing the 3D model of the dental object based on the plurality of adjusted 3D scan points for each of the plurality of identified 3D scan points.

FIG. 1

**Description**

**FIELD**

The disclosure relates to a method and an intraoral scanning system that is configured to optimize a three-dimensional model of a dental object. More specifically, the disclosure relates to an improved way of removing motion artifacts, such as motion blurring and motion warping, in two-dimensional images used for generating a three-dimensional model.

**BACKGROUND**

Motion blur and motion warp compensation are generally known from three-dimensional (3D) intraoral scanners, and it remains a problem to provide motion blur and/or motion warp compensation in complex scanner scenarios where the warp of the intraoral scanner while performing a 3D scanning of a dental object is varying more than normal. In the more complex scanner scenarios known motion blur or motion warp compensation methods would not be able to compensate the warp in the two-dimensional images such that that a human eye would not be able to address it. EP2654606B1 discloses a computer implemented method for compensating motion blur when performing a 3D scanning of at least a part of an object by means of an intraoral scanner. The motion blur occurs because the scanner and the object are moved relative to each other during the acquisition of two-dimensional images at different focus planes or at different times. The motion warp occurs because different parts of the image is composed of multiple images, as is the cased with focus scanning. In another case, a camera with a rolling shutter instead of a global shutter would create motion warp when moving the camera while capturing images. The warp between the different two-dimensional images is compensated by determine different transformation matrices between the different two-dimensional images or for each of the two-dimensional images. In a complex scanner scenario where the motion between points in a two-dimensional image varies differently then applying the same transformation matrix to all points would not be able to compensate for the different motions between the points.

**SUMMARY**

It is an aspect of the present disclosure to overcome the above-mentioned disadvantage of compensating for motion blur and motion warp in a three-dimensional model.
Motion and warp are used interchangeably throughout the description. Furthermore, warp/motion is a general term which covers motion blur and motion warp.
According to the aspect, an intraoral scanning system is disclosed. The intraoral scanner system may be configured for optimizing a three-dimensional (3D) model of a dental object for motion blur. The system may include an intraoral scanner configured to capture a plurality of intraoral scans of a dental object, and wherein each of the plurality of intraoral scans includes a plurality of 3D scan points. The plurality of intraoral scans may be captured based on a scanning principle such as triangulation-based scanning, confocal scanning, focus scanning, or optical coherent tomography OCT. In focus scanning the plurality of intraoral scans may correspond to sub-scans which include a combination of pixels in focus of acquired two-dimensional images at different focus planes during a focus sweep. In triangulation-based scanning, the plurality of intraoral scans may correspond to sub-scans which include pixels in focus.
A single intraoral scan may be obtained by capturing a number of two-dimensional images, i.e. a plurality of sub-scans, at different positions of a focus plane. As the focus plane coincides with the scan surface, a pattern will be projected onto a surface point in-focus and with high contrast. The high contrast gives rise to a large spatial variation of the pattern on the surface of the object, thereby providing a large variation, or amplitude, of the pixel values over a group of adjacent pixels. For each group of pixels, it is thus possible to identify individual settings of the focusing plane for which each group of pixels will be in focus. By using knowledge of the optical system used, it is possible to transform the contrast information vs. position of the focus plane into 3D scan points, on an individual pixel group basis. The pattern may be a time-varying or a static pattern.
The 2D to 3D conversion of the image data can be performed in several ways known in the art. I.e. the 3D scan points may be determined by finding the focus plane corresponding to the maximum light oscillation amplitude for each pixel of the 2D image, or for each group of pixels, when recording light amplitude for a range of different focus planes. In some embodiments, the focus plane is adjusted in equal steps from one end of the scanning region to the other. The focus plane may be moved in a range large enough to at least coincide with the surface of the dental object being scanned.
The system may include one or more processors configured to receive the plurality of intraoral scans, and the one or more processors may be configured to receive a 3D model that is determined by a plurality of intraoral scans of a dental object captured by an intraoral scanner or by averaging a plurality of intraoral scans of a dental object captured by an intraoral scanner, wherein each of the plurality of intraoral scans includes a plurality of 3D scan points. Averaging the plurality of intraoral scan would reduce any motion blur which appear in the plurality of intraoral scans. Each of the plurality of intraoral scans includes a plurality of 3D scan points. Furthermore, the one or more processors may be configured to identify a plurality of 3D scan points of a first intraoral scan of the plurality of intraoral scans and to determine a plurality of corresponding sample 3D scan points of the 3D model by selecting each of the plurality of corresponding sample 3D scan points in the 3D model that is closest to a 3D scan point of the identified plurality of 3D scan points. The selection of the plurality of corresponding sample 3D scan points is based on a proximity approach. The one or more processors may be configured to determine, based on a plurality of transformation matrices, a plurality of adjusted 3D scan points for each of the plurality of identified 3D scan points, by minimizing a distance between each of the plurality of identified 3D scan points relative to each of the plurality of corresponding sample 3D scan points for each of the plurality of transformation matrices. Furthermore, the one or more processors may be configured to assign different weighting coefficients to the plurality of adjusted 3D scan points for each of the plurality of identified 3D scan points and to optimize the 3D model of the dental object based on the plurality of adjusted 3D scan points for each of the plurality of identified 3D scan points. The weighting coefficients determine the impact of each of the plurality of transformation matrices to the plurality of adjusted 3D scan points which is an advantage when compensating motion, more specifically, compensating for warp motion in each of the plurality of identified 3D scan points that varies significantly. Each of the plurality of transformation matrices is adapted to different motions, more specifically, to warp motions.

According to the aspect, a computer implemented method for determining an optimized three-dimensional model of a dental object is disclosed. The method may comprise receiving a 3D model that is determined by stitching a plurality of intraoral scans of a dental object captured by an intraoral scanner, wherein each of the plurality of intraoral scans includes a plurality of 3D scan points. Furthermore, the method may comprise identifying a plurality of 3D scan points of a first intraoral scan of the plurality of intraoral scans, determining a plurality of corresponding sample 3D scan points of the 3D model by selecting each of the plurality of corresponding sample 3D scan points in the 3D model that is closest to a 3D scan point of the identified plurality of 3D scan points, and determining, based on a plurality of transformation matrices, a plurality of adjusted 3D scan points for each of the plurality of identified 3D scan points, by minimizing a distance between each of the plurality of identified 3D scan points relative to each of the plurality of corresponding sample 3D scan points for each of the plurality of transformation matrices. The method may further comprise assigning different weighting coefficients to the plurality of adjusted 3D scan points for each of the plurality of identified 3D scan points, and optimizing the 3D model of the dental object based on the plurality of adjusted 3D scan points for each of the plurality of identified 3D scan points.

The optimization of the 3D model may be based on a summation of the plurality of adjusted 3D scan points for each of the plurality of identified 3D scan points. Which means that a single adjusted 3D scan point may be impacted differently by the plurality of transformation matrices depending on the different weighting coefficients. Furthermore, each of the plurality of identified 3D scan points is assigned to a different weighting coefficient The different weighting coefficients may be determined by a timestamp of each of the plurality of identified 3D scan points. Alternatively, the different weighting coefficients may be determined by depth data retrieved from the plurality of intraoral scans, color brightness of the dental object that is determined by the plurality of intraoral scans etc.

Each of the plurality of 3D scan points may be captured by the intraoral scanner at a point-in-time. The point-in-time may be a timestamp. Thus, each of the different weighting coefficients may be determined based on the captured point-in-time of each of the plurality of identified 3D scan points.

Since the value of a weighting coefficient of the different weighting coefficients is depended on a timestamp/point-in-time of the corresponding identified 3D scan point, it would be possible to adjust the impact of each of the plurality of transformation matrices to the adjusted 3D scan point depending on when the corresponding 3D scan point was captured, i.e. depending on the timestamp/point-in-time of the corresponding 3D scan point.

The different weighting coefficients may be interrelated based on a common weighting coefficient that is determined based on the captured point-in-time of each of the plurality of identified 3D scan points. The different weighting coefficients may be determined based on a Bernstein polynomials. The different weighting coefficients $(b_1, b_2)$ may include following weighting coefficients:

$$b_1(t_i) = \omega_i(t_i) \, ; \; b_2(t_i) = 1 - \omega_i(t_i)$$

, where $\omega_i$ is the common weighting coefficient. The common weighting coefficient may be determined by following equation:

$$\omega_i(t_i) = \frac{t_i - t_{min}}{t_{max} - t_{min}}$$

wherein i represents an index of identified 3D scan point of the plurality of identified 3D scan points, t represents the captured point-in-time of the identified 3D scan point, tmin represents a minimum captured point-in-time of the plurality of identified 3D scan points and tmax represents a maximum captured point-in-time of the plurality of identified 3D scan points. The minimum and maximum point-in-time (tmin, tmax) may be determined over a period of identified intraoral scans of the plurality of identified intraoral scans.

The different weighting coefficients $b_1$, $b_2$, $b_3$, $b_4$ may be determined by following equations depending on a number (N) of different transformation matrices (T) of the plurality of transformation matrices:

For N=2:

$$b_1 \tfrac{1}{2}(t_i) = \omega_i(t_i) \; ; \; b_2(t_i) = 1 - \omega_i(t_i)$$

For N=3:

$$b_1(t_i) = (1 - \omega_i(t_i))^2; \; b_2(t_i) = 2\omega_i(t_i)\bigl(1 - \omega_i(t_i)\bigr); \; b_3(t_i) = \omega_i^2(t_i)$$

For N=4:

$$b_1(t_i) = (1 - \omega_i(t_i))^3; \; b_2(t_i) = 3\omega_i(t_i)(1 - \omega_i(t_i))^2; \; b_3(t_i)$$
$$= 3\omega_i^2(t_i)\bigl(1 - \omega_i(t_i)\bigr); \; b_4(t_i) = \omega_i^3(t_i)$$

. The number of different transformation matrices corresponds to the number of different weighting coefficients. The different weighting coefficients ($b_1,b_2,b_3$) may be denoted as weighting functions where at a given time, $t_i$, the weighting function becomes a weighting coefficient, and the weighting function outputs different weighting coefficients at different time, $t_i$.

The minimizing of the distance between each of the plurality of identified 3D scan points (pi) relative to each of the plurality of corresponding sample 3D scan points (Si) for each of the plurality of transformation matrices may be based on an iterative closest point (IPC) algorithm.

The method may further comprise determining a level of motion distortion in the plurality of 3D scan points (p) of each of the plurality of intraoral scans, and determining, based on the level of motion distortion, a number of different transformation matrices in the plurality of transformation matrices. The number of different transformation matrices that are needed to optimally compensate for motion blur in the plurality of 3D scan points depends on how different the motion in the plurality of identified 3D scan points turns out. For example, the one or more processors may be configured to perform a motion analysis of the plurality of identified 3D scan points for then to determine, based on the motion analysis, the number of different transformation matrices of the plurality of different transformation matrices. The motion analysis may be based on a statistical analysis of how much the motion varies of the plurality of identified 3D scan points. The statistical analysis may be based on standard deviation, variance, spread, or dispersion. The motion analysis includes one or more statistical thresholds for standard deviation, variance, spread, or dispersion. By being able to optimize the number of different transformation matrices would prevent unnecessary adjustments of the 3D scan points if having included too many unnecessary transformation matrices. Thus, if using too few different transformation matrices would result in less optimal motion compensation. The number of transformation matrices may be determined during a post-processing step relative to the processing of the 3D model based on the plurality of intraoral scans. The number of transformation matrices may be a pre-processing step relative to the determination of the plurality of adjusted 3D scan points.

The optimization of the 3D model for motion blur may appear as a post-processing step to the rendering of the 3D model in real time while scanning the dental object. The optimized 3D model may be displayed in a non-real matter after the rendering of the complete 3D model has been finalized. The displaying of the optimized 3D model in non-real-time may appear when receiving a user input.

The optimization of the 3D model for motion blur and/or motion warp may appear as a in real time while the 3D model of the dental object is being stitched or rendered. The 3D model may be displayed and updated in real-time.

The dental object may include one or more teeth, gingiva or tissue inside a mouth of a patient.

The 3D model may be determined by the plurality of intraoral scans that has been acquired by an intraoral scanner. Each of the plurality of intraoral scans includes a plurality of 3D scan points and/or a plurality of adjusted 3D scan points that has been optimized by the plurality of transformation matrices. For producing the 3D model, the plurality of intraoral scans are stitched together based on an overlap between neighbouring intraoral scans of the plurality of intraoral scans. Which means that two neighbouring intraoral scans overlaps partially the same area of a dental object being scanned. The 3D model of the dental object may be optimized by a global optimization algorithm which applies an adjustment of the 3D scan points that forms part of the overlap between two neighbouring intraoral scans. The adjustment of the 3D scan points of a first overlap would inevitably affect the adjustment of the 3D scan points of a second overlap and soon, which means, the adjustment involves the 3D scan points of all overlaps between neighbouring intraoral scans of the plurality of intraoral scans. The global optimization algorithm may perform the adjustment of the 3D scan points sequentially, simultaneously or randomly.

The 3D scan points of an overlap between two neighbouring intraoral scans includes a first group of 3D scan points of a first intraoral scan and a second group of 3D scan points of a second intraoral scan. The differences between the first and second group of 3D scan points are minimized in such a way that the absolute changes applied to the first and second group of 3D scan points would be the same. Which results in adjustments, provided by the global optimization algorithm, that avoids applying any adjustments to the 3D scan points that are more affected by either the first or the second group of 3D scan points in an overlap.

The global optimization algorithm is either performed before or after the 3D model is being optimized by the plurality of adjusted 3D scan points.

According to the aspect, a further computer implemented method for determining an optimized three-dimensional (3D) model of a dental object is disclosed. The method comprising receiving a 3D model that is determined by a plurality of intraoral scans of a dental object captured by an intraoral scanner, wherein each of the plurality of intraoral scans includes a plurality of 3D scan points. The plurality of intraoral scans includes a first intraoral scan and a second intraoral scan, wherein the first and second intraoral scans include 3D scan points which overlaps an area of the dental object, and the first intraoral scan includes a first group of 3D scan points which overlaps a second group of 3D scan points of the second intraoral scan. The differences between the first and second group of 3D scan points are minimized, by a global optimization algorithm, in such a way that the absolute changes applied to the first and second group of 3D scan points would be the same.

Before or after the applying of the global optimization algorithm, a plurality of corresponding sample 3D scan points of the 3D model is determined by selecting each of the plurality of corresponding sample 3D scan points in the 3D model that is closest to a 3D scan point of the identified plurality of 3D scan points. Furthermore, based on a plurality of transformation matrices, a plurality of adjusted 3D scan points for each of the plurality of identified 3D scan points is determined by minimizing a distance between each of the plurality of identified 3D scan points relative to each of the plurality of corresponding sample 3D scan points for each of the plurality of transformation matrices. Additionally, different weighting coefficients are assigned to the plurality of adjusted 3D scan points for each of the plurality of identified 3D scan points ($p_i$), and the 3D model of the dental object is optimized based on the plurality of adjusted 3D scan points for each of the plurality of identified 3D scan points.

## BRIEF DESCRIPTION OF THE FIGURES

Aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features and/or technical effect will be apparent from and elucidated with reference to the illustrations described hereinafter in which:

FIG. 1 illustrates an example of a computer implantable method,
FIG. 2 illustrates another example of a computer implantable method,
FIG. 3 illustrates yet another example of a computer implantable method,
FIG. 4 illustrates an example of motion compensation based on a plurality of different transformation matrices,
FIG. 5 illustrates another example of a computer implantable method,
FIG. 6 illustrates an example of selection of a plurality of transformation matrices,
FIG. 7 illustrates an example of displaying a 3D model and an optimized 3D model,
FIG. 8 illustrates an example of an intraoral scanner system, and
FIGS. 9A, 9B and 9C illustrate an example of a global optimization algorithm.

## DETAILED DESCRIPTION

The detailed description set forth below in connection with the appended drawings is intended as a description of various configurations. The detailed description includes specific details for the purpose of providing a thorough understanding of various concepts. However, it will be apparent to those skilled in the art that these concepts may be practiced without these specific details. Several aspects of the devices, systems, mediums, programs and methods are described by various blocks, functional units, modules, components, circuits, steps, processes, algorithms, etc. (collectively referred to as "elements"). Depending upon particular application, design constraints or other reasons, these elements may be implemented using electronic hardware, computer program, or any combination thereof.

The electronic hardware may include microprocessors, microcontrollers, digital signal processors (DSPs), field programmable gate arrays (FPGAs), programmable logic devices (PLDs), gated logic, discrete hardware circuits, and other suitable hardware configured to perform the various functionality described throughout this disclosure. Computer program shall be construed broadly to mean instructions, instruction sets, code, code segments, program code, programs, subprograms, software modules, applications, software applications, software packages, routines, subroutines, objects, executables, threads of execution, procedures, functions, etc., whether referred to as software, firmware, middleware, microcode, hardware description language, or otherwise.

A scanning for providing intra-oral scan data may be performed by a dental scanning system that may include an intraoral scanning device such as the TRIOS series scanners from 3 Shape A/S. The dental scanning system may include a wireless capability as provided by a wireless network unit. The scanning device may employ a scanning principle such as triangulation-based scanning, confocal scanning, focus scanning, ultrasound scanning, x-ray scanning, stereo vision, structure from motion, optical coherent tomography OCT, or any other scanning principle. In an embodiment, the scanning device is capable of obtaining surface information by operated by projecting a pattern and translating a focus plane along an optical axis of the scanning device and capturing a plurality of 2D images at different focus plane positions such that each series of captured 2D images corresponding to each focus plane forms a stack of 2D images. The acquired 2D images are also referred to herein as raw 2D images, wherein raw in this context means that the images have not been subject to image processing. The focus plane position is preferably shifted along the optical axis of the scanning system, such that 2D images captured at several focus plane positions along the optical axis form said stack of 2D images (also referred to herein as a sub-scan) for a given view of the object, i.e. for a given arrangement of the scanning system relative to the object. After moving the scanning device relative to the object or imaging the object at a different view, a new stack of 2D images for that view may be captured. The focus plane position may be varied by means of at least one focus element, e.g., a moving focus lens. The scanning device is generally moved and angled relative to the dentition during a scanning session, such that at least some sets of sub-scans overlap at least partially, to enable reconstruction of the digital dental 3D model by stitching overlapping 3D sub-scans together in real-time and display the progress of the virtual 3D model on a display as feedback to the user. The result of stitching is the digital 3D representation of a surface larger than that which can be captured by a single sub-scan, i.e. which is larger than the field of view of the 3D scanning device. Stitching by identifying overlapping regions of 3D surface in various sub-scans and transforming sub-scans to a common coordinate system such that the overlapping regions match, finally yielding the digital 3D model. An Iterative Closest Point (ICP) algorithm may be used for this purpose. Another example of a scanning device is a triangulation scanner, where a time varying pattern is projected onto the dental object and a sequence of images of the different pattern configurations are acquired by one or more cameras located at an angle relative to the projector unit.

Color texture of the dental object may be acquired by illuminating the object using different monochromatic colors such as individual red, green and blue colors or my illuminating the object using multichromatic light such as white light. A 2D image may be acquired during a flash of white light.

Generally, the process of obtaining surface information in real time of a dental object to be scanned requires the scanning device to illuminate the surface and acquire high number of 2D images. Typically, a high-speed camera is used with a framerate of 300-2000 2D frames pr second dependent on the technology and 2D image resolution. The high amount of image data needed to be handled by the scanning device to eighter directly forward the raw image data stream to an external processing device or performing some image processing before transmitting the data to an external device or display. This process requires that multiple electronic components inside the scanner is operating with a high workload thus requiring a high demand of current.

The scanning device comprises one or more light projectors configured to generate an illumination pattern to be projected on a three-dimensional dental object during a scanning session. The light projector(s) preferably comprises a light source, a mask signal having a spatial pattern, and one or more lenses such as collimation lenses or projection lenses. The light source may be configured to generate light of a single wavelength or a combination of wavelengths (mono- or polychromatic). The combination of wavelengths may be produced by using a light source configured to produce light (such as white light) comprising different wavelengths. Alternatively, the light projector(s) may comprise multiple light sources such as LEDs individually producing light of different wavelengths (such as red, green, and blue) that may be combined to form light comprising the different wavelengths. Thus, the light produced by the light source may be defined by a wavelength defining a specific color, or a range of different wavelengths defining a combination of colors such as white light. In an embodiment, the scanning device comprises a light source configured for exciting fluorescent material of the teeth to obtain fluorescence data from the dental object. Such a light source may be configured to produce a narrow range of wavelengths. In another embodiment, the light from the light source is infrared (IR) light, which is capable of penetrating dental tissue. The light projector(s) may be DLP projectors using a micro mirror array for generating a time varying pattern, or a diffractive optical element (DOF), or back-lit mask signal projectors, wherein the light source is placed behind a mask signal having a spatial pattern, whereby the light projected on the surface of the dental object is patterned. The back-lit mask signal projector may comprise a collimation lens for collimating the light from the light source, said collimation lens being placed between the light source and the mask signal. The mask signal may have a checkerboard pattern, such that the generated illumination pattern is a checkerboard pattern. Alternatively, the mask signal may feature other patterns such as lines or dots, etc.

The scanning device preferably further comprises optical components for directing the light from the light source to the surface of the dental object. The specific arrangement of the optical components depends on whether the scanning device is a focus scanning apparatus, a scanning device using triangulation, or any other type of scanning device. A focus scanning apparatus is further described in EP 2 442 720 B1 by the same applicant, which is incorporated herein in its entirety.

The light reflected from the dental object in response to the illumination of the dental object is directed, using optical components of the scanning device, towards the image sensor(s). The image sensor(s) are configured to generate a plurality of images based on the incoming light received from the illuminated dental object. The image sensor unit may be a high-speed image sensor such as an image sensor configured for acquiring images with exposures of less than 1/1000 second or frame rates in excess of 250 frames pr. second (fps). As an example, the image sensor may be a rolling shutter (CCD) or global shutter sensor (CMOS). The image sensor(s) may be a monochrome sensor including a color filter array such as a Bayer filter and/or additional filters that may be configured to substantially remove one or more color components from the reflected light and retain only the other non-removed components prior to conversion of the reflected light into an electrical signal. For example, such additional filters may be used to remove a certain part of a white light spectrum, such as a blue component, and retain only red and green components from a signal generated in response to exciting fluorescent material of the teeth.

The network unit may be configured to connect the dental scanning system to a network comprising a plurality of network elements including at least one network element configured to receive the processed data. The network unit may include a wireless network unit or a wired network unit. The wireless network unit is configured to wirelessly connect the dental scanning system to the network comprising the plurality of network elements including the at least one network element configured to receive the processed data. The wired network unit is configured to establish a wired connection between the dental scanning system and the network comprising the plurality of network elements including the at least one network element configured to receive the processed data.

The dental scanning system preferably further comprises a processor configured to generate scan data (such as extra-oral scan data and/or intra-oral scan data) by processing the two-dimensional (2D) images acquired by the scanning device. The processor may be part of the scanning device. As an example, the processor may comprise a Field-programmable gate array (FPGA) and/or an Advanced RISC Machines (ARM) processor located on the scanning device. The scan data comprises information relating to the three-dimensional dental object. The scan data may comprise any of: 2D images, 3D point clouds, depth data, texture data, intensity data, color data, and/or combinations thereof. As an example, the scan data may comprise one or more point clouds, wherein each point cloud comprises a set of 3D points describing the three-dimensional dental object. As another example, the scan data may comprise images, each image comprising image data e.g. described by image coordinates and a timestamp $(x, y, t)$, wherein depth information can be inferred from the timestamp. The image sensor(s) of the scanning device may acquire a plurality of raw 2D images of the dental object in response to illuminating said object using the one or more light projectors. The plurality of raw 2D images may also be referred to herein as a stack of 2D images. The 2D images may subsequently be provided as input to the processor, which processes the 2D images to generate scan data. The processing of the 2D images may comprise the step of determining which part of each of the 2D images are in focus in order to deduce/generate depth information from the images. The internal depth information may be used to generate 3D point clouds comprising a set of 3D points in space, e.g., described by cartesian coordinates $(x, y, z)$. The 3D point clouds may be generated by the processor or by another processing unit. Each 2D/3D point may furthermore comprise a timestamp that indicates when the 2D/3D point was recorded, i.e., from which image in the stack of 2D images the point originates. The timestamp is correlated with the z-coordinate of the 3D points, i.e., the z-coordinate may be inferred from the timestamp. Accordingly, the output of the processor is the scan data, and the scan data may comprise image data and/or depth data, e.g. described by image coordinates and a timestamp $(x, y, t)$ or alternatively described as $(x, y, z)$. The scanning device may be configured to transmit other types of data in addition to the scan data. Examples of data include 3D information, texture information such as infra-red (IR) images, fluorescence images, reflectance color images, x-ray images, and/or combinations thereof.

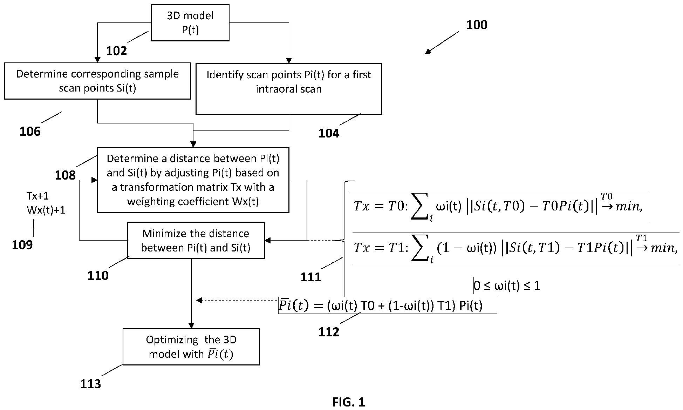

FIG. 1 illustrates an example of a computer implemented method 100 for determining an optimized three-dimensional (3D) model of a dental object. The method 100 comprises receiving 102 a 3D model that is determined by averaging a plurality of intraoral scans P(t) of a dental object captured by an intraoral scanner, wherein each of the plurality of intraoral scans includes a plurality of 3D scan points, wherein each of the plurality of intraoral scans includes a plurality of 3D scan points(p(t)). Thus, the method 100 includes identifying 104 a plurality of 3D scan points (pi(t)) of a first intraoral scan of the plurality of intraoral scans and determining 106 a plurality of corresponding sample 3D scan points Si(t) of the 3D model by selecting each of the plurality of corresponding sample 3D scan points Si(t) in the 3D model that is closest to a 3D scan point of the identified plurality of 3D scan points. Then the method 100 includes determining 108, based on a plurality of transformation matrices (T0, T1, 111), a plurality of adjusted 3D scan points ($\overline{Pi}(t)$, 112) for each of the plurality of identified 3D scan points (pi(t)), by minimizing 110 a distance between each of the plurality of identified 3D scan points (pi) relative to each of the plurality of corresponding sample 3D scan points (Si) for each of the plurality of transformation matrices. In this specific example illustrated in FIG. 1, the method includes assigning different weighting coefficients 109 to the plurality of adjusted 3D scan points $\overline{Pi}(t)$, for each of the plurality of identified 3D scan points (pi(t)), and optimizing 113 the 3D model of the dental object based on the plurality of adjusted 3D scan points $\overline{P_i}(t)$ for each of the plurality of identified 3D scan points (pi(t)). In this specific example illustrated in FIG. 1 the weighting coefficient ($\omega$i(t), 1-$\omega$i(t)) is dependent on time. In another example, the weighting coefficeints may be based on Bernstein polynomials (b1, b2, b3, b4).

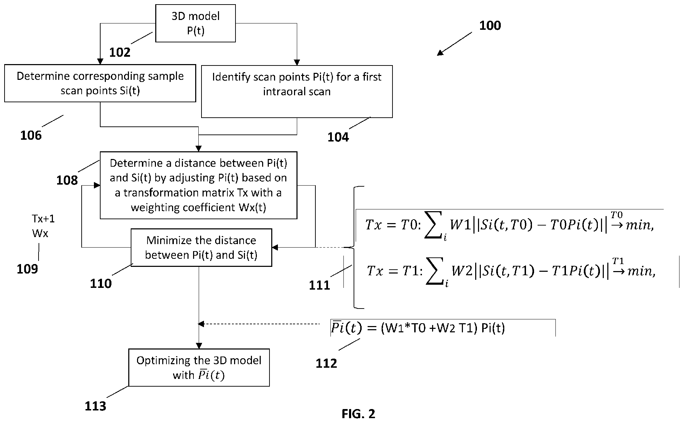

FIG. 2 illustrates an example of a computer implemented method 100 for determining an optimized three-dimensional (3D) model of a dental object. The method 100 comprises receiving 102 a 3D model that is determined by averaging a plurality of intraoral scans of a dental object captured by an intraoral scanner, wherein each of the plurality of intraoral scans includes a plurality of 3D scan points (P(t)). Thus, the method 100 includes identifying 104 a plurality of 3D scan points (pi(t)) of a first intraoral scan of the plurality of intraoral scans and a plurality of corresponding sample 3D scan points Si(t) of the 3D model by selecting each of the plurality of corresponding sample 3D scan points Si(t) in the 3D model that is closest to a 3D scan point of the identified plurality of 3D scan points Pi(t). Then the method 100 includes determining 108, based on a plurality of transformation matrices (T0, T1, 111), a plurality of adjusted 3D scan points ($\overline{Pi}(t)$, 112) for each of the plurality of identified 3D scan points (pi(t)), by minimizing 110 a distance between each of the plurality of identified 3D scan points (pi) relative to each of the plurality of corresponding sample 3D scan points (Si) for each of the plurality of transformation matrices. In this specific example illustrated in FIG. 1, the method includes assigning different weighting coefficients 109 to the plurality of adjusted 3D scan points $\overline{Pi}(t)$, for each of the plurality of identified 3D scan points (pi(t)), and optimizing 113 the 3D model of the dental object based on the plurality of adjusted 3D scan points $\overline{Pi}(t)$ for each of the plurality of identified 3D scan points (pi(t)). In this specific example illustrated in FIG. 1 the weighting coefficients (W1,W2) are not time depended but are determined by a weighting coefficient algorithm that has been trained by intraoral scan data from previous intraoral scans, and wherein the intraoral scan data includes motion blur which have been compensated by the weighting coefficients determined by the weighting coefficient algorithm. The weighting coefficient algorithm may include one or more of the following deep learning architecture:

- Convolutional neural network,
- Recurrent neural network,
- Template matching, and
- Eigenfaces.

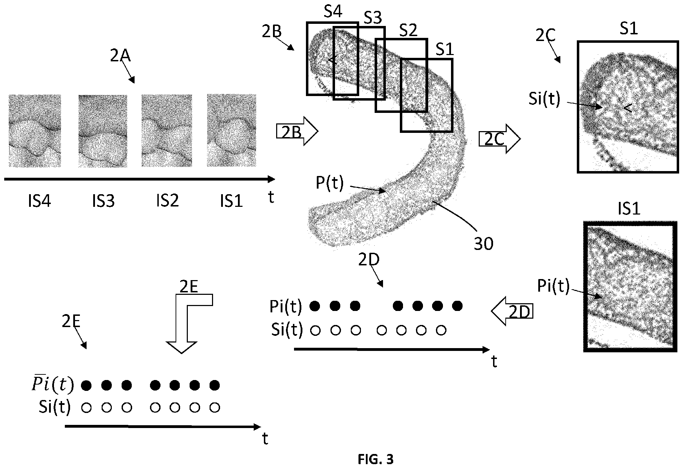

FIG. 3 illustrates an example of the method 100 compensating motion blur in an intraoral scan (S1, S2, S3, S4). In step 2A, a sequence of a plurality of intraoral scans (IS1, IS2, IS3, IS4) is acquired by an intraoral scanner (not shown) which is stitched together to generate a 3D model 30, see step 2B. Motion blur that may exist in the plurality of intraoral scans P(t) would be reduced by averaging the plurality of intraoral scans P(t) in the 3D model 30. In this example, the 3D model 30 is illustrated as a mesh of plurality of 3D scan points P(t), where each of the plurality of 3D scan points P(t) has a timestamp, e.g. a point-in-time that indicates when that 3D scan point P(t) has been captured by the intraoral scanner. In step 2C, a plurality of corresponding sample 3D scan points Si(t) is determined by selecting each of the plurality of corresponding sample 3D scan points (Si(t),S1) in the 3D model that is closest to a 3D scan point of the identified plurality of 3D scan points (Pi(t),IS1). In 2D, the timing of the scan points of the identified plurality of 3D scan points Pi(t) and the plurality of corresponding sample 3D scan points Si(t) is seen, and is clearly seen a mismatch in the timing and that motion blur is more pronounced in the identified plurality of 3D scan points Pi(t). In Step 2E, the method adjusts $\overline{Pi}(t)$ the plurality of identified 3D scan points Pi(t) relative to the plurality of sample 3D scan points Si(t) based on the plurality of transformation matrices (T0,T1) with/without assigned weighting coefficients.

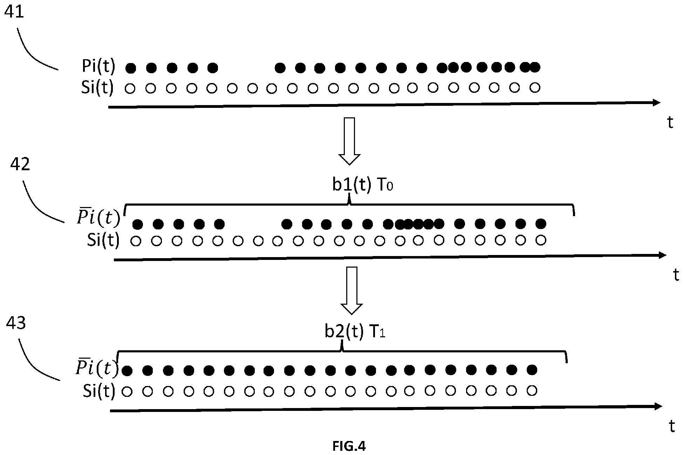

FIG. 4 illustrates the plurality of transformation matrices (T0,T1) and the different weighting coefficients ($\omega$i(t), (1-$\omega$i(t))) being assigned to the plurality of identified 3D scan points Pi(t). The different weighting coefficients ($b_1$, $b_2$) include following weighting coefficients:

$$b_1(t_i) = \omega_i(t_i) \; ; \; b_2(t_i) = 1 - \omega_i(t_i)$$

, where $\omega_i$ is the common weighting coefficient. The common weighting coefficient is determined by following equation:

$$\omega_i(t_i) = \frac{t_i - t_{min}}{t_{max} - t_{min}}$$

wherein i represents an index of identified 3D scan point of the plurality of identified 3D scan points, t represents the captured point-in-time of the identified 3D scan point, tmin represents a minimum captured point-in-time of the plurality of identified 3D scan points and tmax represents a maximum captured point-in-time of the plurality of identified 3D scan points. The minimum and maximum point-in-time (tmin, tmax) may be determined over a period of identified intraoral scans of the plurality of identified intraoral scans.

In the example illustrated in FIG. 4, the movement of the intraoral scanner during scanning 41 has impacted a severe variation in the motion between each of the plurality of identified 3D scan points Pi(t). In the example, the plurality of transformation matrices includes two transformation matrices ($T_0$, $T_1$), such as a first transformation matrix $T_0$ and a second transformation matrix $T_1$. The impact of each of the plurality of transformation matrices is scaled to a different weighting coefficient $b_1(t)$, $b_2(t)$, in this example, the first transformation matrix $T_0$ is scaled to $b_1(t)$ and the second transformation matrix $T_1$ is scaled to $b_2(t)$. This means that when a 3D scan point Pi(t) is captured later in time the first transformation matrix T0 has an increasing impact to the adjustment of the 3D scan points $\overline{Pi}(t)$, however, the impact of the second transformation T1 to the adjustment of 3D scan points $\overline{Pi}(t)$ becomes less. In 42 it is seen that the later captured 3D scan points Pi(t) have mainly been adjusted by the first transmission matrix T0, and in 43 the earlier captured 3D scan points have mainly been adjusted by the second transmission matrix T1.

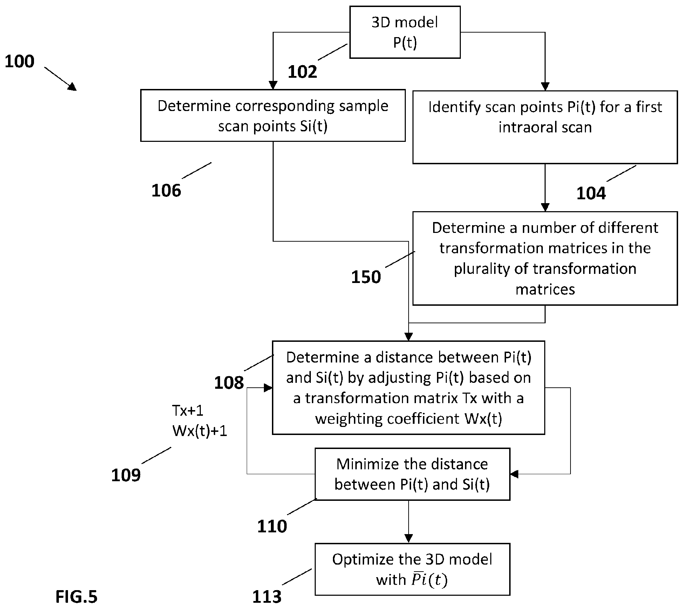

FIG. 5 illustrates an example of a computer implemented method 100 for determining an optimized three-dimensional (3D) model of a dental object. The method 100 comprises receiving 102 a 3D model that is determined by a plurality of intraoral scans of a dental object captured by an intraoral scanner, wherein each of the plurality of intraoral scans includes a plurality of 3D scan points P(t). Thus, the method 100 includes identifying 104 a plurality of 3D scan points (Pi(t)) of a first intraoral scan of the plurality of intraoral scans. To know the complexity of the motion warp that has been applied to the 3D model a a motion warp analysis of the plurality of identified 3D scan points Pi(t) is to be carry out. Then a number of different transformation matrices of the plurality of different transformation matrices is determined 150 based on the motion warp analysis. In another example, the number of different transformation matrices of the plurality of different transformation matrices is determined 150 is determined by an eigenvalue analysis of the plurality of different transformation matrices. Furthermore, the method 100 includes determining a plurality of corresponding sample 3D scan points Si(t) of the 3D model by selecting each of the plurality of corresponding sample 3D scan points Si(t) in the 3D model that is closest to a 3D scan point of the identified plurality of 3D scan points Pi(t). Then the method 100 includes determining 108, based on a plurality of transformation matrices (T0, T1, 111), a plurality of adjusted 3D scan points ($\overline{Pi}(t)$, 112) for each of the plurality of identified 3D scan points (pi(t)), by minimizing 110 a distance between each of the plurality of identified 3D scan points (pi) relative to each of the plurality of corresponding sample 3D scan points (Si) for each of the plurality of transformation matrices. In this specific example illustrated in FIG. 5, the method includes assigning different weighting coefficients 109 to the plurality of adjusted 3D scan points $\overline{Pi}(t)$, for each of the plurality of identified 3D scan points (pi(t)), and optimizing 113 the 3D model of the dental object based on the plurality of adjusted 3D scan points $\overline{Pi}(t)$ for each of the plurality of identified 3D scan points (pi(t)).

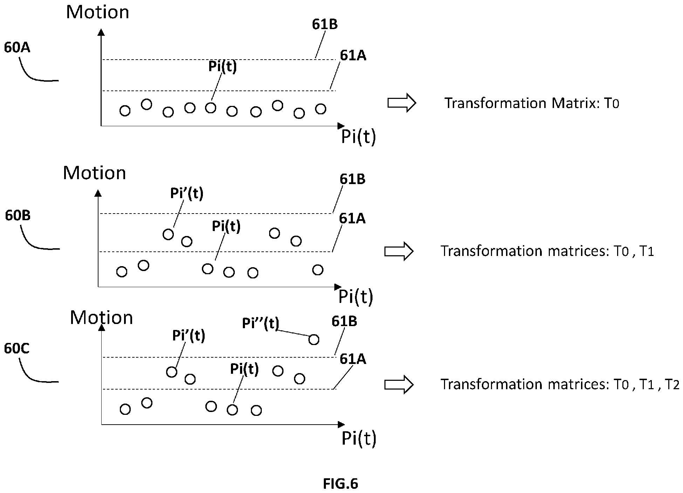

FIG. 6. illustrates an example of a motion analysis which is used to determine the number of different transformation matrices ($T_0$, $T_1$, $T_2$) to be used for compensating for the motion in the plurality of identified 3D scan points Pi(t). The motion analysis may be performed by the one or more processors. In the examples (60A,60B,60C) the one or more processors perform a motion analysis of the variance of the motion of the plurality of identified 3D scan points Pi(t). The motion analysis may be based on a statistical analysis of how much the motion varies of the plurality of identified 3D scan points. The statistical analysis may be based on standard deviation, variance, spread, or dispersion. The motion analysis includes one or more statistical thresholds for standard deviation, variance, spread, or dispersion. When correcting for motion warp in a plurality of 3D scan points at a time, it could be beneficial to constrain the optimization of the 3D model by requiring that a group of 3D scan points that are close in time to have the same motion warp. So, instead of performing the optimization of the 3D model by assigning a weighting coefficient to each of the 3D scan points in the group, the optimization of the 3D model may include assigning the same weighting coefficient to the group of 3D scan points. Thereby, it is not needed to determine and assign a weighting coefficient for each of the 3D scan points. In the examples illustrated in FIG. 6, a first 61A and a second 61B statistical threshold are predetermined. In example 60A, the motion of the plurality of identified 3D scan points Pi(t) are below the first statistical threshold 61A which will result in a single transformation matrix to be applied for adjusting the plurality of identified 3D scan points Pi(t). In example 60B, the motion of a group Pi'(t) of identified 3D scan points is above the first threshold and another group Pi(t) of identified 3D scan points is below the first threshold, which results in that the plurality of different transformation matrices includes two transformation matrices (T0, T1). In example 60C, the motion of the identified 3D scan points defines three groups of identified 3D scan points (Pi(t), Pi'(t), Pi"(t)), such as a first group of identified 3D scan points Pi(t) that is below the first statistical threshold 61A, a second group of identified 3D scan points Pi'(t) that is above the first statistical threshold 61A and below the second statistical threshold 61B, and a third group of identified 3D scan points Pi''(t) that is above the second statistical threshold 61B. The motion analysis of the identified scan points (Pi(t),Pi'(t), Pi" (t)) results in which results in that the plurality of different transformation matrices includes three transformation matrices (T0, T1, T2).

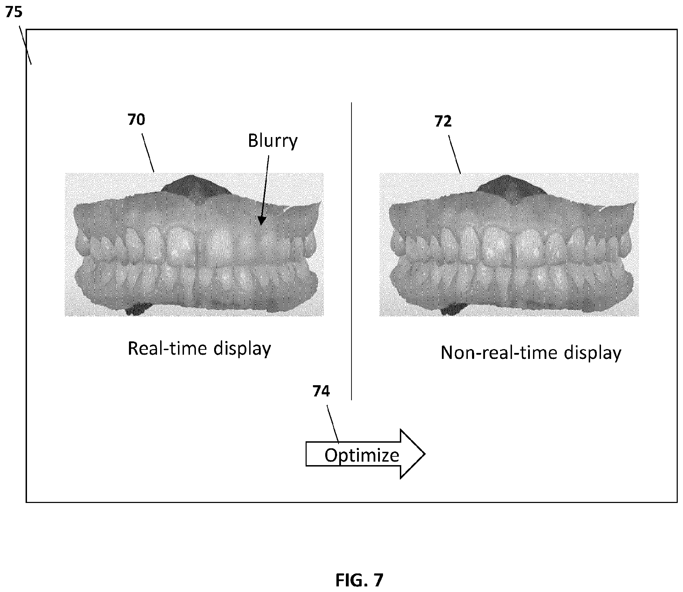

FIG. 7 illustrates a workflow that includes displaying 75 the 3D model 70 in a real time manner during scanning of a dental object. After the completion of the 3D model 70 a post processing step including at least the method 100 may be initiated upon receiving a user input 74. After or during the post-processing step an optimized 3D model (72,113) is displayed. In this example, the optimized 3D model (72,113 is being displayed in non-real time manner. In Fig. 7 it is seen that a part of the un-optimized 3D model 70 is blurry, and the blurry part is removed in the optimized 3D model 72.

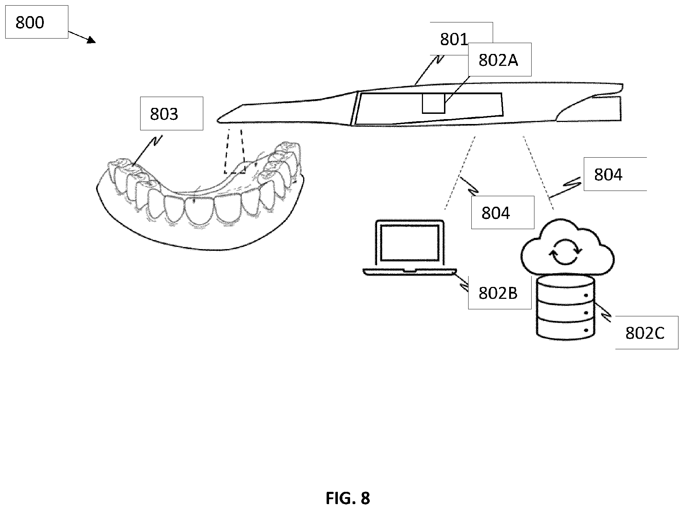

FIG. 8 illustrates an intraoral scanner system 800 that is configured to optimize a three-dimensional (3D) model 70 of a dental object. The system 800 includes an intraoral scanner 801 configured to capture a plurality of intraoral scans of a dental object 803, and wherein each of the plurality of intraoral scans includes a plurality of 3D scan points(P). In this example, the system 800 includes one or more processors (802A,802B,802C) that is configured to perform the method 100. In this example the method may be processed by one or all of the processors (802A, 802B, 802C). In this example, the one or more processors includes a first processor 802A that is arranged within the intraoral scanner 801 , a second processor 802B arranged in an external computer, and a third processor 802C that is arranged in a cloud or a regular server 802C.

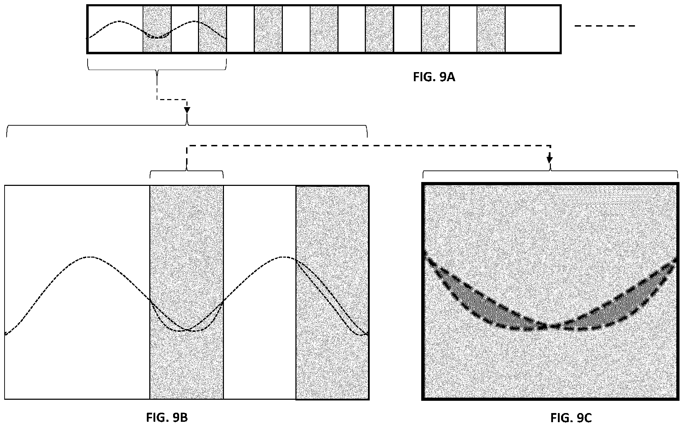

FIGS. 9A, 9B and 9C illustrate a global optimization algorithm a global optimization algorithm which applies an adjustment of the 3D scan points that forms part of the overlap (91) between two neighbouring intraoral scans (S1, S2). The adjustment of the 3D scan points of a first overlap would inevitably affect the adjustment of the 3D scan points of a second overlap S2 and soon, which means, the adjustment involves the 3D scan points of all overlaps between neighbouring intraoral scans of the plurality of intraoral scans. The global optimization algorithm may perform the adjustment of the 3D scan points sequentially, simultaneously or randomly. The global optimization algorithm is either performed before or after the 3D model is being optimized by the plurality of adjusted 3D scan points. The 3D scan points of an overlap 91 between two neighbouring intraoral scans (S1, S2) includes a first group of 3D scan points of a first intraoral scan S1 and a second group of 3D scan points of a second intraoral scan S2. The differences between the first and second group of 3D scan points are minimized in such a way that the absolute changes applied to the first and second group of 3D scan points would be the same. Which results in adjustments, provided by the global optimization algorithm, that avoids applying any adjustments to the 3D scan points that are more affected by either the first or the second group of 3D scan points in an overlap.

Many modifications and other embodiments of the inventions set forth herein will come to mind of one skilled in the art to which these inventions pertain having the benefit of the teachings presented in the foregoing descriptions and the associated drawings. Therefore, it is to be understood that the inventions are not to be limited to the specific embodiments disclosed and that modifications and other embodiments are intended to be included within the scope of the appended claims. Moreover, although the foregoing descriptions and the associated drawings describe example embodiments in the context of certain example combinations of elements and/or functions, it should be appreciated that different combinations of elements and/or functions may be provided by alternative embodiments without departing from the scope of the appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

**ITEM**

1. A computer implemented method for determining an optimized three-dimensional (3D) model of a dental object, wherein the method comprising:

- receiving a 3D model that is determined by a plurality of intraoral scans of a dental object captured by an intraoral scanner, wherein each of the plurality of intraoral scans includes a plurality of 3D scan points,
- identifying a plurality of 3D scan points of a first intraoral scan of the plurality of intraoral scans,
- determining a plurality of corresponding sample 3D scan points of the 3D model by selecting each of the plurality of corresponding sample 3D scan points in the 3D model that is closest to a 3D scan point of the identified plurality of 3D scan points,
- determining, based on a plurality of transformation matrices, a plurality of adjusted 3D scan points for each of the plurality of identified 3D scan points, by minimizing a distance between each of the plurality of identified 3D scan points relative to each of the plurality of corresponding sample 3D scan points for each of the plurality of transformation matrices,
- assigning different weighting coefficients to the plurality of adjusted 3D scan points for each of the plurality of identified 3D scan points (pi), and
- optimizing the 3D model of the dental object based on the plurality of adjusted 3D scan points for each of the plurality of identified 3D scan points.

2. The method according to item 1, wherein the optimization of the 3D model is based on a summation of the plurality of adjusted 3D scan points for each of the plurality of identified 3D scan points.

3. The method according to any of the previous items, wherein each of the plurality of 3D scan points is captured at a point-in-time.

4. The method according to item 3, wherein each of the different weighting coefficients are determined based on the captured point-in-time of each of the plurality of identified 3D scan points.

5. The method according to any of the previous items, wherein the minimizing of the distance between each of the plurality of identified 3D scan points relative to each of the plurality of corresponding sample 3D scan points for each of the plurality of transformation matrices is based on an iterative closest point algorithm.

6. The method according to any of the previous items, comprising determining a level of motion distortion in the plurality of 3D scan points of each of the plurality of intraoral scans, and determining, based on the level of motion distortion, a number of different transformation matrices in the plurality of transformation matrices.

7. The method according to any of the previous items, wherein the different weighting coefficients are interrelated based on a common weighting coefficient that is determined based on the captured point-in-time of each of the plurality of identified 3D scan points.

8. The method according to item 7, wherein the common weighting coefficient is determined by following equation:

$$\omega_i = \frac{t_i - t_{min}}{t_{max} - t_{min}},$$

wherein i represents an index of identified 3D scan point of the plurality of identified 3D scan points, $t$ represents the captured point-in-time of the identified 3D scan point, $t_{min}$ represents a minimum captured point-in-time of the plurality of identified 3D scan points and $t_{max}$ represents a maximum captured point-in-time of the plurality of identified 3D scan points within a single intraoral scan of the plurality of intraoral scan.

9. The method according to item 8, wherein the different weighting coefficients are determined by following equations depending on a number (N) of different transformation matrices of the plurality of transformation matrices:

For N=2:

$$b_1(t_i) = \omega_i(t_i) \; ; \; b_2(t_i) = 1 - \omega_i(t_i)$$

For N=3:

$$b_1(t_i) = (1 - \omega_i(t_i))^2; \; b_2(t_i) = 2\omega_i(t_i)\big(1 - \omega_i(t_i)\big); \; b_3(t_i) = \omega_i^2(t_i)$$

For N=4:

$$b_1(t_i) = (1 - \omega_i(t_i))^3; \; b_2(t_i) = 3\omega_i(t_i)(1 - \omega_i(t_i))^2; \; b_3(t_i)$$
$$= 3\omega_i^2(t_i)\big(1 - \omega_i(t_i)\big); \; b_4(t_i) = \omega_i^3(t_i)$$

, wherein N represents the number of different transformation matrices, $b_1$, $b_2$, $b_3$, $b_4$ are the different weighting coefficients and $\omega_i$ is the common weighting coefficient.

10. The method according to any of the previous items, comprising displaying the 3D model in real-time while scanning the dental object with the intraoral scanner, and displaying the optimized 3D model in non-real-time.

11. The method according to any of the previous items, comprising displaying the optimized 3D model in non-real-time when receiving a user input.

12. An intraoral scanner system configured for optimizing a three-dimensional (3D) model of a dental object, comprising:

- an intraoral scanner configured to capture a plurality of intraoral scans of a dental object, and wherein each of the plurality of intraoral scans includes a plurality of 3D scan points,
- one or more processors configured to receive the plurality of intraoral scans, and wherein the one or more processors is configured to:

  o receive a 3D model that is determined by averaging a plurality of intraoral scans of a dental object captured by an intraoral scanner, wherein each of the plurality of intraoral scans includes a plurality of 3D scan points,
  o identify a plurality of 3D scan points of a first intraoral scan of the plurality of intraoral scans,
  o determine a plurality of corresponding sample 3D scan points of the 3D model by selecting each of the plurality of corresponding sample 3D scan points in the 3D model that is closest to a 3D scan point of the identified plurality of 3D scan points,
  o determine, based on a plurality of transformation matrices, a plurality of adjusted 3D scan points for each of the plurality of identified 3D scan points, by minimizing a distance between each of the plurality of identified 3D scan points relative to each of the plurality of corresponding sample 3D scan points for each of the plurality of transformation matrices,
  o assign different weighting coefficients to the plurality of adjusted 3D scan points for each of the plurality of identified 3D scan points (pi), and

  optimizing the 3D model of the dental object based on the plurality of adjusted 3D scan points for each of the plurality of identified 3D scan points.

13. The system according to item 14, wherein the optimization of the 3D model is based on a summation of the plurality of adjusted 3D scan points for each of the plurality of identified 3D scan points.

14. The system according to any of items 12 and 13, wherein each of the plurality of 3D scan points is captured at a point-in-time.

15. The system according to item 14, wherein each of the different weighting coefficients are determined, by the one or more processors, based on the captured point-in-time of each of the plurality of identified 3D scan points.

16. The system according to any of items 12 to 15, wherein the minimization of the distance between each of the plurality of identified 3D scan points relative to each of the plurality of corresponding sample 3D scan points for each of the plurality of transformation matrices is based on an iterative closest point algorithm.

17. A computer implemented method for determining an optimized three-dimensional (3D) model of a dental object, wherein the method comprising:

- receiving a 3D model that is determined by averaging a plurality of intraoral scans of a dental object captured by an intraoral scanner, wherein each of the plurality of intraoral scans includes a plurality of 3D scan points,
- identifying a plurality of 3D scan points of a first intraoral scan of the plurality of intraoral scans,
- determining a plurality of corresponding sample 3D scan points of the 3D model by selecting each of the plurality of corresponding sample 3D scan points in the 3D model that is closest to a 3D scan point of the identified plurality of 3D scan points,
- determining, based on a plurality of transformation matrices, a plurality of adjusted 3D scan points for each of the plurality of identified 3D scan points, by minimizing a distance between each of the plurality of identified 3D scan points relative to each of the plurality of corresponding sample 3D scan points for each of the plurality of transformation matrices,
- assigning different weighting coefficients to the plurality of adjusted 3D scan points for each of the plurality of identified 3D scan points (pi), and
- optimizing the 3D model of the dental object based on the plurality of adjusted 3D scan points for each of the plurality of identified 3D scan points.

**Claims**

1. A computer implemented method for determining an optimized three-dimensional (3D) model of a dental object, wherein the method comprising:

• receiving a 3D model that is determined by a plurality of intraoral scans of a dental object captured by an intraoral scanner, wherein each of the plurality of intraoral scans includes a plurality of 3D scan points,
• identifying a plurality of 3D scan points of a first intraoral scan of the plurality of intraoral scans,
• determining a plurality of corresponding sample 3D scan points of the 3D model by selecting each of the plurality of corresponding sample 3D scan points in the 3D model that is closest to a 3D scan point of the identified plurality of 3D scan points,
• determining, based on a plurality of transformation matrices, a plurality of adjusted 3D scan points for each of the plurality of identified 3D scan points, by minimizing a distance between each of the plurality of identified 3D scan points relative to each of the plurality of corresponding sample 3D scan points for each of the plurality of transformation matrices,
• assigning different weighting coefficients to the plurality of adjusted 3D scan points for each of the plurality of identified 3D scan points (pi), and
• optimizing the 3D model of the dental object based on the plurality of adjusted 3D scan points for each of the plurality of identified 3D scan points.

2. The method according to claim 1, wherein the optimization of the 3D model is based on a summation of the plurality of adjusted 3D scan points for each of the plurality of identified 3D scan points.

3. The method according to any of the previous claims, wherein each of the plurality of 3D scan points is captured at a point-in-time.

4. The method according to claim 3, wherein each of the different weighting coefficients are determined based on the captured point-in-time of each of the plurality of identified 3D scan points.

5. The method according to any of the previous claims, wherein the minimizing of the distance between each of the plurality of identified 3D scan points relative to each of the plurality of corresponding sample 3D scan points for each of the plurality of transformation matrices is based on an iterative closest point algorithm.

6. The method according to any of the previous claims, comprising determining a level of motion distortion in the plurality of 3D scan points of each of the plurality of intraoral scans, and determining, based on the level of motion distortion, a number of different transformation matrices in the plurality of transformation matrices.

7. The method according to any of the previous claims, wherein the different weighting coefficients are interrelated based on a common weighting coefficient that is determined based on the captured point-in-time of each of the plurality of identified 3D scan points.

8. The method according to claim 7, wherein the common weighting coefficient is determined by following equation:

$$\omega_i = \frac{t_i - t_{min}}{t_{max} - t_{min}},$$

wherein i represents an index of identified 3D scan point of the plurality of identified 3D scan points, $t$ represents the captured point-in-time of the identified 3D scan point, $t_{min}$ represents a minimum captured point-in-time of the plurality of identified 3D scan points and $t_{max}$ represents a maximum captured point-in-time of the plurality of identified 3D scan points within a single intraoral scan of the plurality of intraoral scan.

9. The method according to claim 8, wherein the different weighting coefficients are determined by following equations depending on a number (N) of different transformation matrices of the plurality of transformation matrices:

For N=2:

$$b_1(t_i) = \omega_i(t_i) \; ; \; b_2(t_i) = 1 - \omega_i(t_i)$$

For N=3:

$$b_1(t_i) = (1 - \omega_i(t_i))^2; \; b_2(t_i) = 2\omega_i(t_i)\big(1 - \omega_i(t_i)\big); \; b_3(t_i) = \omega_i^2(t_i)$$

For N=4:

$$b_1(t_i) = (1 - \omega_i(t_i))^3; \ b_2(t_i) = 3\omega_i(t_i)(1 - \omega_i(t_i))^2; \ b_3(t_i)$$
$$= 3\omega_i^2(t_i)\big(1 - \omega_i(t_i)\big); \ b_4(t_i) = \omega_i^3(t_i)$$

, wherein N represents the number of different transformation matrices, $b_1$, $b_2$, $b_3$, $b_4$ are the different weighting coefficients and $\omega_i$ is the common weighting coefficient.

10. An intraoral scanner system configured for optimizing a three-dimensional (3D) model of a dental object, comprising:

- an intraoral scanner configured to capture a plurality of intraoral scans of a dental object, and wherein each of the plurality of intraoral scans includes a plurality of 3D scan points,
- one or more processors configured to receive the plurality of intraoral scans, and wherein the one or more processors is configured to:

o receive a 3D model that is determined by a plurality of intraoral scans of a dental object captured by an intraoral scanner, wherein each of the plurality of intraoral scans includes a plurality of 3D scan points,
o identify a plurality of 3D scan points of a first intraoral scan of the plurality of intraoral scans,
o determine a plurality of corresponding sample 3D scan points of the 3D model by selecting each of the plurality of corresponding sample 3D scan points in the 3D model that is closest to a 3D scan point of the identified plurality of 3D scan points,
o determine, based on a plurality of transformation matrices, a plurality of adjusted 3D scan points for each of the plurality of identified 3D scan points, by minimizing a distance between each of the plurality of identified 3D scan points relative to each of the plurality of corresponding sample 3D scan points for each of the plurality of transformation matrices,
o assign different weighting coefficients to the plurality of adjusted 3D scan points for each of the plurality of identified 3D scan points (pi), and

optimizing the 3D model of the dental object based on the plurality of adjusted 3D scan points for each of the plurality of identified 3D scan points.

3D model
P(t)

**100**

**102**

Determine corresponding sample
scan points Si(t)

Identify scan points Pi(t) for a first
intraoral scan

**106**

**104**

Determine a distance between Pi(t)
and Si(t) by adjusting Pi(t) based on
a transformation matrix Tx with a
weighting coefficient Wx(t)

**108**

Tx+1
Wx(t)+1

**109**

Minimize the distance
between Pi(t) and Si(t)

**110**

**111**

$$Tx = T0: \sum_i \omega i(t) \, \big| \big| Si(t, T0) - T0Pi(t) \big| \big| \overset{T0}{\to} min,$$

$$Tx = T1: \sum_i (1 - \omega i(t)) \, \big| \big| Si(t, T1) - T1Pi(t) \big| \big| \overset{T1}{\to} min,$$

$$0 \le \omega i(t) \le 1$$

$$\overline{Pi}(t) = (\omega i(t) \, T0 + (1 - \omega i(t)) \, T1) \, Pi(t)$$

**112**

Optimizing the 3D
model with $\overline{Pi}(t)$

**113**

FIG. 1

**3D model P(t)**

**102**

**Determine corresponding sample scan points Si(t)**

**Identify scan points Pi(t) for a first intraoral scan**

**100**

**106**

**104**

**Determine a distance between Pi(t) and Si(t) by adjusting Pi(t) based on a transformation matrix Tx with a weighting coefficient Wx(t)**

**108**

**Tx+1 Wx**

**Minimize the distance between Pi(t) and Si(t)**

**109**

**110**

**111**

$$Tx = T0: \sum_i W1\left|\left|Si(t,T0) - T0Pi(t)\right|\right|\overset{T0}{\rightarrow} min,$$

$$Tx = T1: \sum_i W2\left|\left|Si(t,T1) - T1Pi(t)\right|\right|\overset{T1}{\rightarrow} min,$$

$$\overline{Pi}(t) = (W1*T0 + W2\ T1)\ Pi(t)$$

**112**

**Optimizing the 3D model with $\overline{Pi}(t)$**

**113**

**FIG. 2**

FIG. 3

FIG.4

**100**

3D model
P(t)

**102**

Determine corresponding sample
scan points Si(t)

Identify scan points Pi(t) for a first
intraoral scan

**106**

**104**

Determine a number of different
transformation matrices in the
plurality of transformation
matrices

**150**

Determine a distance between Pi(t)
and Si(t) by adjusting Pi(t) based on
a transformation matrix Tx with a
weighting coefficient Wx(t)

**108**

Tx+1
Wx(t)+1

**109**

Minimize the distance
between Pi(t) and Si(t)

**110**

Optimize the 3D model
with $\overline{Pi}(t)$

**FIG.5**                **113**

FIG.6

**75**

**70**
Blurry

**72**

Real-time display

Non-real-time display

**74**

Optimize

**FIG. 7**

**FIG. 8**

**FIG. 9A**

**FIG. 9B**

**FIG. 9C**

Europäisches
Patentamt

European
Patent Office

Office européen
des brevets

## EUROPEAN SEARCH REPORT

**Application Number**

EP 25 18 4837

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2021/353154 A1 (SAPHIER OFER [IL] ET AL) 18 November 2021 (2021-11-18) <br> * paragraph [0062] * <br> * paragraph [0226] - paragraph [0229] * <br> * paragraphs [0314], [0523] * | 1-10 | INV. <br> G06T5/73 <br> G06T7/55 |
| A | WO 2024/119138 A2 (ALIGN TECHNOLOGY INC [US]) 6 June 2024 (2024-06-06) <br> * the whole document * | 1-10 | |
| A | WO 2023/014995 A1 (ALIGN TECHNOLOGY INC [US]) 9 February 2023 (2023-02-09) <br> * the whole document * | 1-10 | |
| A | JP 2015 527896 A (3SHAPE AS) 24 September 2015 (2015-09-24) <br> * paragraph [0202] * | 1-10 | |
| A | AHN JAE SUNG ET AL: "A three-dimensional scanning apparatus based on structured illumination method and its application in dental scanning", <br> PROCEEDINGS OF SPIE; [PROCEEDINGS OF SPIE ISSN 0277-786X VOLUME 10524], SPIE, US, vol. 10373, 23 August 2017 (2017-08-23), pages 103730T-103730T, XP060096550, DOI: 10.1117/12.2271018 ISBN: 978-1-5106-1533-5 <br> * page 5 * | 1-10 | TECHNICAL FIELDS SEARCHED (IPC) <br><br> G06T |
| A | US 2019/191141 A1 (FISKER RUNE [DK] ET AL) 20 June 2019 (2019-06-20) <br> * paragraph [0002] * <br> * paragraph [0129] - paragraph [0131] * <br> * paragraph [0193] * | 1-10 | |

-/--

The present search report has been drawn up for all claims

| Place of search | Date of completion of the search | Examiner |
|---|---|---|
| Munich | 22 October 2025 | Fischer, Jan |

page 1 of 2

Europäisches
Patentamt

European
Patent Office

Office européen
des brevets

# EUROPEAN SEARCH REPORT

**Application Number**

EP 25 18 4837

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KR 102 310 662 B1 (HDX WILL CORP [KR]) 12 October 2021 (2021-10-12) * claims 6-7;- * * paragraph [0024] - paragraph [0026] * ----- | 1-10 | |
| A | DENG YINGYAN ET AL: "Root Canal Therapy Assisted Robot System", 2023 WRC SYMPOSIUM ON ADVANCED ROBOTICS AND AUTOMATION (WRC SARA), IEEE, 19 August 2023 (2023-08-19), pages 91-96, XP034433637, DOI: 10.1109/WRCSARA60131.2023.10261785 [retrieved on 2023-09-27] * Section II.B * ----- | 1-10 | |
| A | SEONGJIN PARK ET AL: "An enhanced method for registration of dental surfaces partially scanned by a 3D dental laser scanning", COMPUTER METHODS AND PROGRAMS IN BIOMEDICINE, vol. 118, no. 1, 1 January 2015 (2015-01-01), pages 11-22, XP055509976, NL ISSN: 0169-2607, DOI: 10.1016/j.cmpb.2014.09.007 * Section Abstract * * Section 2.2 * ----- | 1-10 | TECHNICAL FIELDS SEARCHED (IPC) |

The present search report has been drawn up for all claims

| Place of search | Date of completion of the search | Examiner |
|---|---|---|
| Munich | 22 October 2025 | Fischer, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                  

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 18 4837

This annex lists the patent family members relating to the patent documents cited in the above-mentioned European search report.
The members are as contained in the European Patent Office EDP file on
The European Patent Office is in no way liable for these particulars which are merely given for the purpose of information.

22-10-2025

| Patent document cited in search report | | | Publication date | Patent family member(s) | | Publication date |
|---|---|---|---|---|---|---|
| US 2021353154 | A1 | | 18-11-2021 | CN | 115884727 A | 31-03-2023 |
| | | | | EP | 4136569 A1 | 22-02-2023 |
| | | | | US | 2021321872 A1 | 21-10-2021 |
| | | | | US | 2021353152 A1 | 18-11-2021 |
| | | | | US | 2021353153 A1 | 18-11-2021 |
| | | | | US | 2021353154 A1 | 18-11-2021 |
| | | | | US | 2025127399 A1 | 24-04-2025 |
| | | | | WO | 2021211871 A1 | 21-10-2021 |
| WO 2024119138 | A2 | | 06-06-2024 | CN | 120641994 A | 12-09-2025 |
| | | | | EP | 4627591 A2 | 08-10-2025 |
| | | | | US | 2024221307 A1 | 04-07-2024 |
| | | | | US | 2024221308 A1 | 04-07-2024 |
| | | | | WO | 2024119138 A2 | 06-06-2024 |
| WO 2023014995 | A1 | | 09-02-2023 | NONE | | |
| JP 2015527896 | A | | 24-09-2015 | CN | 104780830 A | 15-07-2015 |
| | | | | EP | 2866644 A1 | 06-05-2015 |
| | | | | EP | 4306075 A2 | 17-01-2024 |
| | | | | ES | 2967677 T3 | 03-05-2024 |
| | | | | JP | 6430934 B2 | 28-11-2018 |
| | | | | JP | 2015527896 A | 24-09-2015 |
| | | | | US | 2015164335 A1 | 18-06-2015 |
| | | | | US | 2019183345 A1 | 20-06-2019 |
| | | | | US | 2019231194 A1 | 01-08-2019 |
| | | | | US | 2019365236 A1 | 05-12-2019 |
| | | | | US | 2023263397 A1 | 24-08-2023 |
| | | | | US | 2024423477 A1 | 26-12-2024 |
| | | | | WO | 2014000745 A1 | 03-01-2014 |
| US 2019191141 | A1 | | 20-06-2019 | CN | 103491897 A | 01-01-2014 |
| | | | | EP | 2654606 A1 | 30-10-2013 |
| | | | | EP | 3915511 A1 | 01-12-2021 |
| | | | | ES | 2884700 T3 | 10-12-2021 |
| | | | | US | 2014022352 A1 | 23-01-2014 |
| | | | | US | 2019191141 A1 | 20-06-2019 |
| | | | | US | 2021160471 A1 | 27-05-2021 |
| | | | | WO | 2012083968 A1 | 28-06-2012 |
| KR 102310662 | B1 | | 12-10-2021 | CN | 116782819 A | 19-09-2023 |
| | | | | EP | 4278954 A1 | 22-11-2023 |
| | | | | KR | 102310662 B1 | 12-10-2021 |
| | | | | US | 2024070882 A1 | 29-02-2024 |
| | | | | WO | 2022154523 A1 | 21-07-2022 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2654606 B1
- EP 2442720 B1